# EUROPEAN PATENT APPLICATION

(11) **EP 3 854 419 A1**
(43) Date of publication of application: **28.07.2021**
(21) Application number: 20153550.7
(22) Date of filing: 24.01.2020
(51) Int. Cl.: A61K 48/00, C12N 15/87, C12N 15/113, C12N 15/63

(54) **DNA CONJUGATE AND METHOD OF TRANSFORMATION OF GENES INTO CELLS**

(71) Applicant: Univerzita Karlova, Lekarska fakulta v Plzni, 11636 Praha 1 (CZ)
(72) Inventor: Hrabák, Jaroslav, 19800 Praha 14 (CZ); Svec, Martin, 32300 Plzen (CZ)
(74) Representative: Hartvichova, Katerina

(57) **Abstract**

The present invention relates to a DNA conjugate for transformation of cells, such as bacterial cells, which comprises a double stranded linear DNA containing a promoter and an open reading frame of at least one gene, wherein a loop structure containing 10 to 50 nucleotides is ligated to each end of the double stranded linear DNA, and wherein at least one transport molecule is bound to at least one loop structure, preferably via an amino-modifier moiety residue. The invention also relates to a method of preparing such DNA conjugate.

Furthermore, the invention provides a method for transformation of a cell, e.g. a bacterial cell, comprising the steps of:
- providing a double stranded linear DNA containing a promoter and an open reading frame of at least one gene;
- subjecting the double stranded linear DNA to ligation of a loop structure containing 10 to 50 nucleotides to each end of the double stranded linear DNA, wherein the loop structure at one end or at each end contains at least one base modified with an amino-modifier moiety;
- reacting the base modified with an amino-modifier moiety with a transport molecule to form a covalent or non-covalent bond to the transport molecule to form a DNA conjugate;
- contacting the said DNA conjugate with cells, to transport the DNA conjugate through the cell membrane into the cells.

## Description

### Field of Art

The invention relates to the technical field of DNA modification for improving its stability and efficient penetration to bacterial cells.

### Background Art

An uptake of exogenous genetic material is a crucial step in biotechnology (gene engineering) and gene therapy. For laboratory purposes, transformation is the most common technique for genetic modification of prokaryotic and some eukaryotic cells (e.g., yeasts). In Gram-negative bacteria, the DNA must cross the outer membrane, periplasmic space and cytoplasmic membrane to pass into the cytosolic department of the cell. In Gram-positive bacteria, the DNA must pass a simple cell wall consisting of cytoplasmic membrane and thick structure of peptidoglycan.
The efficiency of DNA transport through the cell membrane can be enhanced by chemical treatment of the cell(s) followed by a heat shock or by electroporation. Another widely used technique is viral transformation where the foreign DNA is translocated into the cell via viral particles (e.g., bacteriophages in bacteria) (Chen I., Dubnau D. 2004. DNA uptake during bacterial transformation. Nat. Rev.Microbiol. 2(3):241-249). For nucleic acid transformation of a cell, several available modifications of the nucleic acid can be used, i.e. plasmid DNA, minicircle DNA, micro-intronic plasmid or RNA (including mRNA or small interfering RNA).
Cell wall of the bacteria is, however, negatively charged, consequently, negatively charged DNA cannot pass that barrier passively (Gigante A., Li M., Junghänel S., Hirschhäuser C., Knauer S., Schmuck C. 2019. Non-Viral Transfection Vectors: Are Hybrid Materials the Way Forward? Med. Chem. Commun. 10.1039/C9MD00275H). For artificial transfection, several transport molecules or systems have been proposed. These systems are covalently or non-covalently bound to the nucleic acid to be transported. DNA can be coupled with a hydrophilic domain, e.g., cationic lipid vectors that can easily pass the phospholipid portion of the membrane; with polymers that bind electrostatically to the negatively charged phosphates, usually high molecular weight phosphates, such polymers include, e.g., polysaccharides (chitosan, dextran), dendrimers, polydimethylaminoethyl methacrylate; with peptides, e.g., cationic fusion peptide; or with other molecules, e.g., cobalamin; or with inorganic nanoparticles, e.g., silver, gold, and iron (Gigante A., Li M., Junghänel S., Hirschhäuser C., Knauer S., Schmuck C. 2019. Non-Viral Transfection Vectors: Are Hybrid Materials the Way Forward? Med. Chem. Commun. 10.1039/C9MD00275H).

### Disclosure of the Invention

The present invention provides a deoxyribonucleic acid (DNA) conjugate for transformation of cells, exhibiting an improved stability and an improved penetration to the cell, which comprises or consists of a double stranded linear DNA containing a promoter and an open reading frame of at least one gene, wherein a loop structure containing 10 to 50 nucleotides is ligated to each end of the double stranded linear DNA, and wherein at least one transport molecule is bound to at least one loop structure, preferably via an amino-modifier moiety residue.

The present invention further provides a method of preparing a deoxyribonucleic acid (DNA) conjugate for transformation of cells, exhibiting an improved stability and an improved penetration to the cell due to coupling with a transport molecule enhancing its transport through the cell membrane, e.g. cobalamin or metallic nanoparticles.

The said methods comprises the following steps:
- providing a double stranded linear DNA containing a promoter and an open reading frame of at least one gene (the gene can encode, e.g., for protein of interest or antisense RNA for expression regulation);
- subjecting the double stranded linear DNA to ligation of a loop structure containing 10 to 50 nucleotides to each end of the double stranded linear DNA, wherein the loop structure at one end or at each end contains at least one base modified (i.e., substituted) with an amino-modifier moiety;
- reacting the base modified with an amino-modifier moiety with a transport molecule to form a covalent or non-covalent bond to the transport molecule.

In a first step of the method, a double stranded linear DNA containing a promoter and an open reading frame is provided. The open reading frame can encode for a gene that can affect the cell, e.g., a gene encoding for a toxin or regulating processes in the cell, or a gene encoding for an antisense RNA that can regulate gene expression, e.g., expression of genes of bacterial toxins or antibiotic resistance genes. Such double stranded linear DNA may be prepared synthetically or by polymerase chain reaction (PCR), typically using primers containing a non-specific sequence on its 5'-end, a restriction site and a sequence specific for the amplification of the gene. The non-specific sequence on the 5'-end of the primer allows for efficient cleavage by a restriction enzyme, and is followed by the sequence specific for the restriction enzyme and by the sequence specific for the gene amplification. If needed, the PCR product or the synthetic preparation product is treated by a restriction enzyme (which is capable of cleaving the respective restriction sites contained in the product) to yield the double stranded linear DNA. When using a PCR amplicon of the target DNA, the restriction sites are designed in the specific oligonucleotides (primers) used for PCR amplification.
Suitable promoters include any constitutive or inducible bacterial promoter, promoter specific for ISEcp1, T7 bacteriophage promoter, promoter from Lac operon, etc.
Both ends of the double stranded linear DNA preferably contain recognition sequences allowing specific ligation of the loop structure to each end.

In a second step of the method, each end of the double stranded linear DNA is subjected to ligation of a loop structure containing 10 to 50 nucleotides wherein preferably 4 to 25 of the nucleotides are unpaired.. Besides the unpaired loop portion, the loop structure contains (at least partially) complementary sequences containing the recognition sequence of a restriction endonuclease that is used for complementary binding to the double-stranded linear DNA. The 5' end of the loop structure can be phosphorylated for efficient ligation to the mentioned linear structure. The loop structure at one end or at each end contains at least one base modified with an amino-modifier moiety.

Amino-modifier moieties are known in the art. Typically, they contain a structure -R-NH-Y, wherein R is a C2-C12 hydrocarbyl (typically C2, C3, C6, C7, C12 hydrocarbyl) in which 1-3 carbons can optionally be replaced by N or substituted by =O, and R may contain 0-2 double bonds; and wherein Y is a protecting group suitable for protecting an amino group, such as trifluoroacetyl or Fmoc, or Y is -(CH₂)ₙ-NH₂, wherein n is 1 to 10, preferably 2 to 6. The amino-modifier moieties are typically bound to the base of a nucleotide (i.e., deoxyribonucleotide) to form a base modified with an amino-modifier moiety. For example, Sigma Aldrich offers the modified nucleotides: Amino-Modifier-C6-dT and Amino-Modifier-C2-dT.

The loop structures on the two ends may be the same or different.

In a third step of the method, the base modified with an amino-modifier moiety is subjected to a reaction with a transport molecule to form a covalent or non-covalent bond to the transport molecule.
Transport molecules are preferably selected from cobalamin, silver nanoparticle, gold nanoparticle, ferric oxide nanoparticle, iron nanoparticle, lipid transport molecules and peptides.
If the amino-modifier group contains Y=PG, then it is de-protected before or during the reaction with the transport molecule

Furthermore, the present invention provides a method for transformation of a cell by a gene, comprising the steps of:
- providing a double stranded linear DNA containing a promoter and an open reading frame of at least one gene;
- subjecting the double stranded linear DNA to ligation of a loop structure containing 10 to 50 nucleotides to each end of the double stranded linear DNA, wherein the loop structure at one end or at each end contains at least one base modified with an amino-modifier moiety;
- reacting the base modified with an amino-modifier moiety with a transport molecule to form a covalent or non-covalent bond to the transport molecule to form a DNA conjugate;
- contacting the said DNA conjugate with the cells, to transport the DNA conjugate through the cell membrane into the cell.

The cells that are transformed are preferably prokaryotic cells, more preferably bacterial cells. More preferably, the cells are selected from causative agents of infectious diseases such as Gram-negative bacteria (e.g. *Enterobacterales, Pseudomonas* spp., *Neisseria* spp., *Acinetobacter* spp., *Haemophilus* spp.), Gram-positive bacteria (e.g., *Staphylococcus aureus, Streptococcus* spp., *Enterococcus* spp., *Listeria monocytogenes*) and acid-fast-staining bacteria (e.g., *Mycobacterium tuberculosis*).

When contacted with the cells, the DNA conjugate is uptaken into the cytoplasm. After incubation of the cells with the DNA conjugate under standard conditions (e.g., 35-37 °C for common bacteria, such as *Escherichia coli* and other *Enterobacterales, Staphylococcus* spp., *Enterococcus* spp., *Mycobacterium* spp.), protein encoded by the gene can be/are expressed by the cells. The system also allows introduction of genes encoding for antisense RNA that can influence protein expression of selected protein(s).

The term "gene" or "gene of interest" means the gene which shall be transported/transfected into the cell (or the cell shall be transformed with the gene). The gene may encode for a protein of interest or for an antisense RNA.

The protein of interest is a protein which shall be produced by the cell after the uptake of the gene. The proteins of interest are typically proteins regulating the cell processes or enzymes or bacterial toxins or antibiotic resistance proteins, but also other types of proteins may be of interest to be produced in the cell. In some embodiments, the protein of interest may be selected from a bacterial toxin that is able to suppress growth of the bacterial cell, a toxin causing lysis of bacterial cell, a protein inhibiting expression of bacterial proteins such as bacterial toxins responsible for virulence, proteins conferring antibiotic resistance.

The antisense RNA typically has the function of regulating expression of genes present in the cell, thereby affecting the expression of the said genes. The encoded RNA may in some embodiments be selected from a small RNA interfering with mRNA forming the RISC complex (RNA-silencing complex) or an RNA cleaving an mRNA.

The term "open reading frame" means the part of a reading frame of a gene that has the ability to be translated. The open reading frame is a continuous sequence of codons which begins with a start codon and ends with a stop codon.

The double stranded linear DNA may contain the open reading frame(s) of one or more genes. It contains two parallel strands joined by hydrogen bonds between complementary purines and pyrimidines.

The term "promoter" designates a region of DNA that leads to initiation of transcription of the open reading frame. Promoter is located upstream of the open reading frame.

The term "loop structure" represents a DNA sequence which contains a loop portion, i.e., a single stranded chain of unpaired nucleotides, and a complementary portion which is composed of two strands, each bound to one end of the loop portion, and the said two strands being mutually complementary in at least part of their sequence. The loop structure typically contains 10 to 50 nucleotides, and its loop portion typically contains 4 to 25 nucleotides.

The loop structure at one end of the conjugate or at both ends of the conjugate contains in the loop portion a nucleotide substituted by a binding moiety, preferably an amino-modifier moiety, through which the transport molecule is bound (covalently or non-covalently).

The term "transport molecule" desginates a molecule enhancing the transport of the DNA conjugate through the cell membrane. It is a molecule that is naturally transported through the membrane, typically via membrane channels or membrane transporters. The transport molecule is preferably selected from cobalamin, lipids, peptides, or metallic nanoparticles.

Metallic nanoparticles are particles with all dimensions smaller than 1 micrometer, more preferably smaller than 500 nm, and typically with dimensions in the range of 1 nm to 1 micrometer, or 1 nm to 500 nm. Preferably, the metallic nanoparticles are made of gold, platinum, silver, ,titanium, iron or oxides or sulfides thereof (e.g., Fe₂O₃). In some embodiments, it may be preferred that the metallic nanoparticles are magnetic which facilitates the synthesis and separation of the conjugate.

### Brief Description of Figures

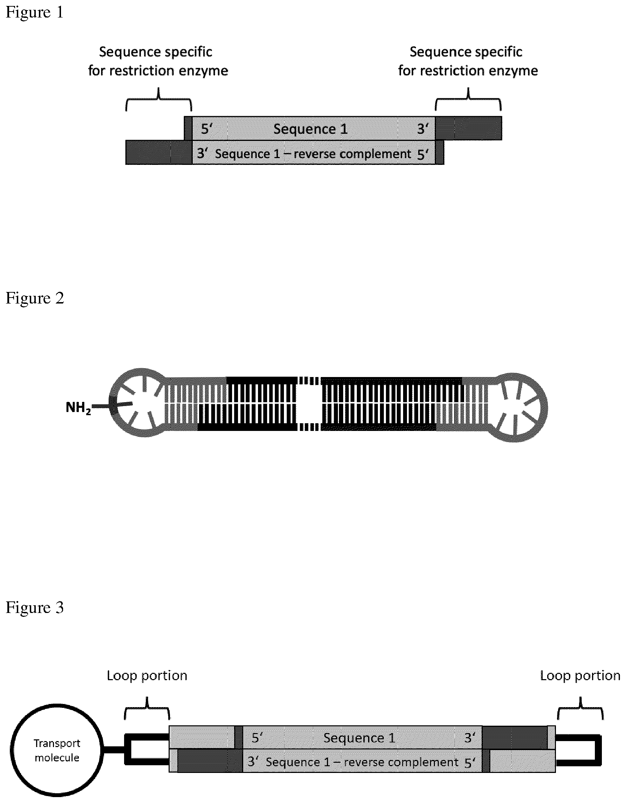

Figure 1 schematically represents the double stranded linear DNA containing a promoter and an open reading frame of a gene of interest.
Figure 2 schematically represents the double stranded linear DNA with the ligated loop structure. The loop structure on one end contains a nucleotide modified with an amino-modifier moiety (only the terminal NH2 group of the amino-modifier moiety is shown).
Figure 3 schematically represents the double stranded linear DNA with the ligated loop structure with a bound transport molecule, i.e., the DNA conjugate of the invention. This DNA conjugate is then further used for transformation of cells.

### Examples

### Example 1: Preparation of DNA fragment containing AmpR-bla_{DHA-1} complex and its transformation to Escherichia coli

The complex of two genes of interest was amplified from the strain of *Klebsiella pneumoniae* containing *bla*_{DHA-1} gene preceded by *ampR* regulator. The promotor of *bla*_{DHA-1} is located in intergenic region of both genes (*bla*_{DHA-1}*, ampR*). For PCR amplification, specific oligonucleotides cttatt**TCTAGA**TTTGATATCGCCTGCCGTGA (forward primer containing Xbal-specific cleavage site - bolded) and acat**GAATTC**AAGCTGTCAGTGCCCGATAC (reverse primer containing EcoRI-specific cleavage site - bolded) were used. The product of the size of ca. 2472 bp was purified by Agencourt® AMPure XP according to the manufacturer's instructions and treated by restriction enzymes Xbal and EcoRI (New England BioLabs Inc.). After digestion, the product was then purified by Agencourt® AMPure XP. The sequences Phosphate-**CTAGA**TGTCTCTCTTTTCCTCC[amino-modifier-C2-dT]CCTCCGTTGTTGTTGTTGAGAGACA**T**, and Phosphate-**AATTC**ATGTCTCTCTTTTCCTCCTCCTCCGTTGTTGTTGTTGAGAGACAT**G** were ligated to the strands (one sequence to each end of the double stranded linear DNA) to form the loop structures.
The structure of amino-modifier-C2-dT (obtained from Sigma-Aldrich) was:

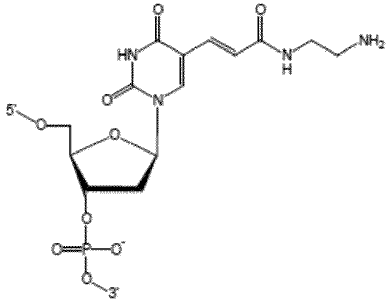

After ligation, the product was purified by ethanol precipitation. Then, a transport molecule -5nm magnetic Fe₂O₃ nanoparticles functionalized with N-hydroxysuccinimide ester (Cat.Nr. 747440, Sigma-Aldrich) were bound to the double stranded DNA with ligated loop structures in 2-(N-morpholino)-ethanesulfonic acid (50 mmol/l, pH 4,5) with incubation at room temperature overnight. The nanoparticles were bound via the amino-modifier-C2 residue. The resulting DNA conjugate was mixed with Tris HCl buffer (50 mmol/l, pH 8,0) and purified using a magnetic stand (Cat.Nr. A31543, ThermoFischer Scientific) and washed two times with Tris HCl buffer (50 mmol/l, pH 8,0). The DNA conjugate was then resuspended in 20 µl of Tris-HCl buffer (50 mmol/l, pH 8,0).
Fifty µl of *Escherichia coli* TOP10 at early exponential phase of growth in Mueller-Hinton broth was incubated with 10 µl of the DNA conjugate of concentration 10 nmol/l at 37 °C. After 1-hour incubation, selection antibiotic (cefoxitin) specifically hydrolyzed by DHA-1 β-lactamase at the concentration of 50 mg/l was added to the culture and incubated another 2 hours. Then, bacteria were harvested by centrifugation and washed 3 times by phosphate buffered saline, total DNA and RNA were extracted and subjected to real-time (quantitative) polymerase chain reaction (PCR) to show the presence of constructs in the cells and reverse-transcription real-time PCR to demonstrate expression of *bla*_{DHA-1}.
Simultaneously, the experiment was conducted also with the double-stranded linear DNA, and with the double-stranded linear DNA with ligated loop structures but without the transport molecule as comparative examples. The results demonstrated high efficiency of transformation using the DNA conjugate of the invention (see Table 1).

**Table 1. PCR results obtained by standard real-time PCR of Escherichia coli TOP10**

| **PCR template** | **Cq (cycle)** |
|---|---|
| Water (negative control) | 41,46 |
| *Escherichia coli* TOP10 (negative control) | 41,39 |
| Double stranded linear DNA (transformed into *Escherichia coli* TOP10) | 40,37 |
| Double stranded linear DNA with ligated loops without transport molecule (transformed into *Escherichia coli* TOP10) | 38,15 |
| Double stranded linear DNA with ligated loops with transport molecule (transformed into *Escherichia coli* TOP10) | 19,76 |
| Double stranded linear DNA - 1000x diluted (positive control) | 16,44 |

### Example 2: Preparation of DNA fragment containing gene bla_{CTX-M-15} preceded by ISEcp1 element and its transformation to Escherichia coli

The gene of interest was bla_{CTX-M-15} gene preceded by *ISEcp1* containing strong promoter, amplified from the strain of *Escherichia coli.* For amplification, specific oligonucleotides cttatt**TCTAGA**ATTGGGTGAAAGAAAAGTGCTCA (forward primer containing Xbal-specific cleavage site - bolded) and acat**GAATTC** ACTTTTGCCGTCTAAGGCGAT (reverse primer containing EcoRI-specific cleavage site - bolded) were used. The product of the size of 2 560 bp was purified by Agencourt® AMPure XP according to the manufacturer's instructions and treated by restriction enzymes Xbal and EcoRI (New England BioLabs Inc.). After digestion, the product was then purified by Agencourt® AMPure XP. The sequences Phosphate-**CTAGA**TGTCTTTATTTTTTTT[amino-modifier-C2-dT]GGGGGGGGTTGAGAGACA**T**, and Phosphate-**AATTC**TGTCTTTATTTTTTTTGGGGGGGGTTGAGAGACAG were ligated to the ends of the strands (each sequence to one end of the double stranded linear product) to form the loop structures. After ligation, the product was purified by ethanol precipitation. Vitamin B12 (cobalamin) was functionalized (carboxylated) by incubation in 1N hydrochloric acid at 37 °C for 4 hrs. Then, the solution was neutralized by adding 0,1N sodium hydroxide to a final pH of 7,5. Carboxylated molecules of vitamin B12 were purified by phenol by addition of equal volume of saturated water solution followed by extraction using acetone/ether solution (1:3) and lyophilized. The carboxylated cobalamin was mixed with the double stranded linear DNA with the ligated loop structures in a ratio of 10:1 in 2-(N-morpholino)-ethanesulfonic acid (50 mmol/l, pH 4,5). Then, 10 mg of *N-*(3-dimethylaminopropyl)-*N*'-ethylcarbodiimide hydrochloride (EDC) was added to the solution and incubated at room temperature overnight. The resulting DNA conjugate was mixed with Tris HCl buffer (50 mmol/l, pH 8,0) and purified by ethanol precipitation. Fifty µl of *Klebsiella pneumoniae* at early exponential phase of growth in Mueller-Hinton broth was incubated at 37 °C with 10 µl of the construct of concentration 10 nmol/l for 3 hours. Then, bacteria were harvested by centrifugation and washed 3 times by phosphate buffered saline. Total DNA and RNA were extracted and subjected to real-time (quantitative) polymerase chain reaction (PCR) to show the presence of the genes in the cells and reverse-transcription real-time PCR to demonstrate expression of *bla*_{CTX-M-15}. Simultaneously, the experiment was conducted also with the double-stranded linear DNA, and with the double-stranded linear DNA with ligated loop structures but without a transport molecule as comparative examples. The results demonstrated a high efficiency of transformation using the DNA conjugate of the present invention (see Table 2).

**Table 2. PCR results obtained by standard real-time PCR of Escherichia coli TOP10**

| **PCR template** | **Cq (cycle)** |
|---|---|
| Water (negative control) | - |
| *Klebsiella pneumoniae* (negative control) | 42,79 |
| Double stranded linear DNA (transformed into *Klebsiella pneumoniae*) | 42,13 |
| Double stranded linear DNA with ligated loops without transport molecule (transformed into *Klebsiella pneumoniae*) | 36,25 |
| Double stranded linear DNA with ligated loops and with transport molecule (transformed into *Klebsiella pneumoniae*) | 13,07 |
| Double stranded linear DNA - 1000× diluted (positive control) | 16,20 |

## Claims

1. DNA conjugate for transformation of cells, which comprises a double stranded linear DNA containing a promoter and an open reading frame of at least one gene, wherein a loop structure containing 10 to 50 nucleotides is ligated to each end of the double stranded linear DNA, and wherein at least one transport molecule is bound to at least one loop structure, preferably via an amino-modifier moiety residue.

2. The DNA conjugate according to claim 1, wherein the gene is a gene encoding for a protein seleected from a bacterial toxin suppressing the growth of the bacterial cell, a toxin causing lysis of bacterial cell, a protein inhibiting expression of bacterial proteins such as bacterial toxins responsible for virulence, proteins conferring antibiotic resistance; or the gene is a gene encoding for an antisense RNA regulating expression of genes.

3. The DNA conjugate according to any one of the preceding claims, wherein the loop structure contains a loop portion, which is a single stranded chain of unpaired nucleotides, and a complementary portion which is composed of two strands, each bound to one end of the loop portion, and the said two strands being mutually complementary in at least part of their sequence.

4. The DNA conjugate according to claim 3 wherein the loop portion contains 4 to 25 nucleotides.

5. The DNA conjugate according to any one of the preceding claims, wherein the transport molecule is selected from cobalamin, lipids, peptides, or metallic nanoparticles.

6. A method of preparing the DNA conjugate according to any one of claims 1 to 5 for transformation of cells, said method comprising the following steps:
- providing a double stranded linear DNA containing a promoter and an open reading frame of at least one gene;
- subjecting the double stranded linear DNA to ligation of a loop structure containing 10 to 50 nucleotides to each end of the double stranded linear DNA, wherein the loop structure at one end or at each end contains at least one base modified with an amino-modifier moiety;
- reacting the base modified with an amino-modifier moiety with a transport molecule to form a covalent or non-covalent bond to the transport molecule.

7. The method according to claim 6, wherein the loop structure contains a loop portion, which is a single stranded chain of unpaired nucleotides, and a complementary portion which is composed of two strands, each bound to one end of the loop portion, and the said two strands being mutually complementary in at least part of their sequence, and wherein the complementary portion contains restriction sites for recognition sequence of a restriction endonuclease.

8. A method for transformation of a cell by a gene using the DNA conjugate according to any one of claims 1 to 5, said method comprising the steps of:
- providing a double stranded linear DNA containing a promoter and an open reading frame of at least one gene;
- subjecting the double stranded linear DNA to ligation of a loop structure containing 10 to 50 nucleotides to each end of the double stranded linear DNA, wherein the loop structure at one end or at each end contains at least one base modified with an amino-modifier moiety;
- reacting the base modified with an amino-modifier moiety with a transport molecule to form a covalent or non-covalent bond to the transport molecule to form a DNA conjugate;
- contacting the said DNA conjugate with cells, to transport the DNA conjugate through the cell membrane into the cells.

9. The method according to claim 8, wherein the cells are selected from Gram-negative bacteria, Gram-positive bacteria and acid-fast-staining bacteria.


SEQUENCE LISTING

<110>  Univerzita Karlova, Lekarska fakulta v Plzni

<120>  DNA conjugate and method of transformation of genes into cells

<130>  P1810

<160>  8

<170>  PatentIn version 3.5

<210>  1
<211>  32
<212>  DNA
<213>  artificial

<220>
<223>  Example 1 - forward primer

<400>  1
cttatttcta gatttgatat cgcctgccgt ga                                    32

<210>  2
<211>  30
<212>  DNA
<213>  artificial

<220>
<223>  Example 1 - reverse primer

<400>  2
acatgaattc aagctgtcag tgcccgatac                                       30

<210>  3
<211>  49
<212>  DNA
<213>  artificial

<220>
<223>  Example 1 - loop structure 1

<400>  3
ctagatgtct ctcttttcct cctcctccgt tgttgttgtt gagagacat                  49

<210>  4
<211>  51
<212>  DNA
<213>  artificial

<220>
<223>  Example 1 - loop structure 2

<400>  4
aattcatgtc tctcttttcc tcctcctccg ttgttgttgt tgagagacat g               51

<210>  5
<211>  35
<212>  DNA

<213>  artificial

<220>
<223>  Example 2 - forward primer

<400>  5
cttatttcta gaattgggtg aaagaaaagt gctca                                35


<210>  6
<211>  31
<212>  DNA
<213>  artificial

<220>
<223>  Example 2 - reverse primer

<400>  6
acatgaattc acttttgccg tctaaggcga t                                    31


<210>  7
<211>  41
<212>  DNA
<213>  artificial

<220>
<223>  Example 1 - loop structure 1

<400>  7
ctagatgtct ttattttttt ttgggggggg ttgagagaca t                         41


<210>  8
<211>  40
<212>  DNA
<213>  artificial

<220>
<223>  Example 2 - loop structure 2

<400>  8
aattctgtct ttattttttt tggggggggt tgagagacag                            40